# FASCICULE DE BREVET EUROPEEN

(11) **EP 1 379 222 B1**
(45) Date de publication et mention de la délivrance du brevet: **10.12.2008**
(21) Numéro de dépôt: 02727667.4
(22) Date de dépôt: 17.04.2002
(51) Int. Cl.: A61K 9/46

(54) **COMPRIMES EFFERVESCENTS ORODISPERSIBLES**
IM MUNDRAUM DISPERGIERENDE BRAUSETABLETTEN
ORODISPERSIBLE EFFERVESCENT TABLETS

(30) Priorité: 20.04.2001 FR 0105389
(43) Date de publication de la demande: 14.01.2004
(73) Titulaire: ETHYPHARM, 78550 Houdan (FR)
(72) Inventeur: DELMAS, Pascal, Outremont, Quebec H2V 3L8 (CA); ZUCCARELLI, Jean-Marc, F-06600 Antibes (FR); MALLARD, Claire, F-06250 Mougins le Haut (FR); MCGINITY, James, W., Austin, TX 78746 (US)
(74) Mandataire: Touati, Catherine
(86) Numéro de dépôt international: PCT/FR2002/001321
(87) Numéro de publication internationale: WO 2002/085336

(56) Documents cités:
- EP-A- 0 737 473
- WO-A-01/80822
- DE-A- 19 822 036
- US-A- 6 071 539

## Description

La présente invention a pour objet des comprimés effervescents orodispersibles.

Au sens de la présente invention, on entend par comprimé orodispersible, un comprimé capable de se désagréger dans la bouche, en l'absence de toute action de mastication, en moins de 60 secondes, de préférence en moins de 40 secondes au contact de la salive en formant une suspension aisée à avaler.

Certains patients, notamment les personnes âgées et les enfants, connaissent des difficultés de déglutition telles qu'il leur est difficile, et par conséquent, désagréable d'ingérer des comprimés ou des gélules, même avec une prise associée de liquide. Ces difficultés ont pour conséquence l'absence de prise du médicament prescrit et ainsi une forte incidence sur l'efficacité du traitement (H. Seager, 1998, J. Pharm. Pharmacol. 50, 375-382).

Or, il est estimé que 50 % de la population connaît de telles difficultés à avaler des comprimés ou des gélules.

Les laboratoires pharmaceutiques ont donc recherché de nouvelles formes pharmaceutiques plus agréables à prendre.

Des comprimés multiparticulaires à délitement rapide ont déjà été décrits par la Société demanderesse dans les brevets FR 99 02516, FR 97 09233, FR 98 14034, FR 92 08642 et FR 91 09245. Ces comprimés comportent des substances actives sous forme de microcristaux enrobés ou de microgranules enrobés, l'enrobage pouvant leur conférer des propriété de masquage de goût, d'amélioration de la stabilité de gastrorésistance ou de libération modifiée du principe actif.

Cependant, compte tenu de la demande toujours croissante en de tels comprimés orodispersibles, il est important de pouvoir présenter de nouvelles formes qui puissent être adaptées à tout type de principe actif et qui soient très faciles et très agréables à prendre.

Les recherches de la Société Demanderesse lui ont permis de trouver, que ces caractéristiques pouvaient être obtenues dès lors que l'on fait comporter des granules effervescents comprenant au moins un principe actif enrobé, obtenus par extrusion à chaud, à un comprimé orodispersible comprenant un mélange d'excipients comprenant au moins un agent de désintégration, un agent soluble et un agent lubrifiant et éventuellement un agent gonflant, un agent perméabilisant, des édulcorants et des arômes.

Les travaux de la Société Demanderesse, décrits dans le brevet US 6.071.539, ont en effet montré que les granules effervescents d'une part facilitent la désagrégation du comprimé et d'autre part lui confèrent des qualités organoleptiques intéressantes, notamment du point de vue du masquage de goût du principe actif, lorsque cela est nécessaire, et de l'obtention de sensations de chatouillements de la cavité buccale, particulièrement appréciées des jeunes enfants.

L'invention a donc pour objet des comprimés effervescents orodispersibles comprenant:
- un mélange d'excipients comprenant au moins un agent de désintégration choisi parmi la croscarmellose, la crospovidone et leur mélange, un agent diluant, un agent lubrifiant et éventuellement un agent déshydratant interne, un agent gonflant, un agent perméabilisant , des édulcorants, des arômes et des colorants,
- et des granules effervescents à base d'au moins un principe actif et d'un mélange constitué d'un agent acide, d'un agent alcalin et d'un liant extrudable à chaud et éventuellement d'un agent lubrifiant et d'un agent déshydratant interne,
lesdits granules étant préparés par extrusion à chaud en l'absence d'eau et de solvants et lesdits comprimés se désagrégeant dans la cavité buccale au contact de la salive en moins de 60 secondes et de préférence en moins de 40 secondes.

Lesdits granules sont préparés par extrusion à chaud en l'absence d'eau et de solvants selon le procédé décrit dans le brevet US 6.071.539.

Le principe actif, incorporé dans les granules effervescents, comporte un enrobage de masquage de goût.

Ce mode de réalisation est particulièrement adapté dans les cas où le principe actif mis en oeuvre présente un goût très désagréable.

L'enrobage qui est alors mis en oeuvre est un enrobage classique de masquage de goût qui peut consister en un polymère ou en un mélange de polymères. Cet enrobage peut en outre présenter des propriétés de stabilisation, de gastrorésistance et de libération modifiée du principe actif.

Les principes actifs mis en oeuvre dans les comprimés conformes à l'invention sont des composés thérapeutiques, des vitamines, des sels minéraux ou des suppléments nutritionnels. Les domaines d'applications de la présente invention peuvent donc être la pharmacie humaine et vétérinaire, et le domaine neutracétique.

Ainsi, on peut utiliser les composés thérapeutiques suivants: les agents antibactériens de synthèse de type acide pyridone-carboxylique peu hydro soluble tel que la benzofloxacine, l'acide nalidixique, l'enoxacine, l'ofloxacine, l'amifloxacine, la fluméquine, la tosfloxacine, l'acide piromidique, l'acide pipémidique, la miloxacine, l'acide oxolinique, la cinoxacine, la norfloxacine, la ciprofloxacine, la perfloxacine, la loméfloxacine, l'enrofloxacine, la danofloxacine, la binfloxacine, la sarafloxacine, l'ibafloxacine, la difloxacine et leurs sels. D'autres composés thérapeutiques qui peuvent être formulés conformément à l'invention comprennent la pénicilline, la tétracycline, l'érythromycine, les céphalosporines et autres antibiotiques.

D'autres principes actifs pouvant être formulés sous forme de comprimés conformes à l'invention comprennent les substances antibactériennes, les antihistamines et les décongestionants, les anti-inflammatoires, les antiparasites, les antiviraux, les agents anxiolytiques, les dérivés de morphine, antagonistes de la sérotonine, les isomères lévogyres de la thyroxine, les agents abaissant le taux de cholestérol, les agents bloquant alpha adrénergiques, les anesthésiques locaux, les antifongiques, les amibicides, ou les agents trichomonocides, les analgésiques, les antiarthritiques, les agents antiasthmatiques, les anticoagulants, les anticonvulsifs, les antidépresseurs, les antidiabétiques, les antinéoplastes, les antipsychotiques, les antihypertenseurs, les dérivés de phénanthrène, les agents antidiarrhéiques, les diurétiques, et les relaxants musculaires.

Comme substances antibactériennes on peut citer les antibiotiques de type bêta-lactam, les tétracyblines, le chloramphénicol, la néomycine, la gramicidine, la bacitracine, les sulfonamides, les antibiotiques de type aminoglycoside, la tobramycine, la nitrofurazone, l'acide nalidixique et leurs analogues et la combinaison antimicrobienne fludalanine/pentizidone. Comme dérivé de phénanthrène, on peut citer le sulfate de morphine. Comme agent antidiarrhéique, on peut citer le chlorhydrate de lopéramide. Comme agent anxiolytique on peut citer la buspirone. Comme agent diurétique, on peut citer l'hydrochlorthiazide. Comme dérivé de morphine, on peut citerle chlorhydrate d'hydromorphone. Comme antihistaminiques et décongestionants, on peut citer la périlamine, la chlorphéniramine, la tétrahydrozoline et l'antazoline. Comme médicaments anti-inflammatoires, on peut citer la cortisone, l'hydrocortisone, la bêtaméthasone, la dexaméthasone, la fluocortolone, la prednisolone, la triamcinolone, l'indométhacinz, le sulindac and ses sels et le sulfure correspondant. Comme composé antiparasite, on peut citer l'ivermectine. Comme composés antiviraux, on peut citer l'acyclovir et l'interferon. Comme médicaments analgésiques, on peut citer le diflunisal, l'aspirine ou l'acétaminophène. Comme antiarthritiques, on peut citer la phénylbutazone, l'indométhacine, le silindac, ses sels et sulfure correspondant, la dexaméthasone, l'ibuprofène, l'allopurinol, l'oxyphenbutazone ou le probenecide. Comme médicaments contre l'asthme, on peut citer la théophylline, l'éphédrine, le dipropionate de beclométhasone et l'épinéphrine. Comme anticoagulants, on peut citer l'héparine, la bishydroxycoumarine, et la warfarine. Comme anticonvulsifs, on peut citer la diphénylhydantoine et le diazépam. Comme antidépresseurs, on peut citer l'amitriptyline, le chlordiazépoxyde la perphénazine, le chlorhydrate de fluoxétine, la protriptyline, l'imipramine et la doxépine. Comme agent abaissant le taux de cholestérol, on peut citer la simvastatine. Comme agent bloquant alpha adrénergique, on peut le tartrate d'ergotamine. Comme antagoniste de la sérotonine, on peut citer le succinate de sumatropine. Comme isomère lévogyre de la thyroxine, on peut citer la lévothyroxine sodique. Comme antidiabétiques, on peut citer, l'insuline, la somatostatine et ses analogues, le tolbutamide, le tolazamide, l'acétchexamide et le chlorpropamide. Comme antinéoplasiques, on peut citer l'adriamycine, le fluorouracil, le méthotrexate et l'asparaginase. Comme antipsychotiques, on peut citer la prochlorpérazine, le carbonate de lithium, le citrate de lithium, la thioridazine, la molindone, la fluphénazine, la trifluopérazine, la perphénazine, l'amitriptyline et la trifluopromazine. Comme antihypertenseurs, on peut citer la spironolactone, la methyldopa, l'hydralazine, la clonidine, le chlorothiazide, la déserpidine, le timolol, le propranolol, le métoprolol, le chlorhydrate de prazosine et la réserpine. Comme relaxants musculaires, on peut citer le succinylcholine-chlorure, la danbrolène, la cyclobenzaprine, le méthocarbamol et le diazepam.

Les composés pharmaceutiques présents dans les comprimés conformes à l'invention peuvent se présenter sous la forme de leurs sels pharmaceutiquement acceptables ou toute forme polymorphe (racémique, énantiomère,...). Par "sels pharmaceutiquement acceptables", on entend les dérivés des composés décrits dans lesquels le composé pharmaceutiquement actif de base est transformé en son sel basique ou acide, des exemples de sels pharmaceutiquement actifs comprennent notamment les sels d'acides organiques ou minéraux de résidus basiques tels que les amines; les dérivés alcalins ou les sels organiques de résidus acides tels que les acides carboxyliques, et similaires. Les sels pharmaceutiquement acceptables comprennent les sels non toxiques classiques ou les sels d'ammonium quaternaire du composé de base, formés par exemple à partir d'acides inorganiques ou organiques non-toxiques. Par exemple, de tels sels non-toxiques classiques comprennent ceux issus d'acides inorganiques tels que chlorhydrique, bromhydrique, sulfurique, sulfonique, sulfamique, phosphorique, nitrique et similaires; et les sels préparés à partir d'acides organiques tels que les acides aminés, acétique, propionique, succinique, glycolique, stéarique, lactique, malique, tartrique, citrique, ascorbique, pamoïque, maléique, hydroxymaléique, phénylacétique, glutamique, benzoïque, salicylique, sulfanilique, 2-acétoxybenzoïque, fumarique, toluènesulfonique, méthanesulfonique, éthane disulfonique, oxalique, isothionique, et similaires.

Les sels pharmaceutiquement acceptables de la présente invention peuvent être synthétisés à partir du composé thérapeutique de base qui contient une fraction acide ou basique, par des procédés classiques. De façon générale, ces sels peuvent être préparés par réaction des formes acides ou basiques libres avec une quantité prédéterminée de la base ou de l'acide approprié dans l'eau ou dans un solvant organique ou dans un mélange d'eau et de solvant organique.

On préfère généralement des milieux non aqueux. On peut trouver des listes de sels appropriés dans Remington's Pharmaceutical Sciences, 17th ed., Mack Publishing Company, Easton, PA, 1985, p. 1418.

L'expression "pharmaceutiquement acceptable" est employée ici pour faire référence aux composés, matières, compositions et/ou formes galéniques qui sont, selon l'appréciation médicale, appropriés pour une utilisation au contact de tissus humains ou animaux sans toxicité, irritation, réponse allergique ou autre problème ou complication excessifs, par rapport à un rapport bénéfice/risque raisonnable.

Le principe actif mis en oeuvre dans les comprimés conformes à l'invention peut également être une vitamine. Tel qu'utilisé ici, le terme vitamine fait référence à toute substance organique à l'état de trace utile dans l'alimentation. Le terme vitamine inclut, notamment la thiamine, la riboflavine, l'acide nicotinique, l'acide pentotènique, la pyridoxine, la biotine, l'acide folique, la vitamine B 12, l'acide lipoique, l'acide ascorbique, la vitamine A, la vitamine D, la vitamine E et la vitamine K. On inclut également sous le terme vitamine leurs coenzymes. Les coenzymes sont des formes chimiques spécifiques des vitamines. Les coenzymes comprennent les thiamine pyrophosphates (TPP), flavine mononucléotide (FMM), flavine adénine dinucléotive (FAD), Nicotinamide adénine dinucléotide (AND), Nicotinamide adénine dinucléotide phosphate (NADP) la coenzyme A (CoA), le pyridoxal phosphate, la biocytine, l'acide tétrahydrofolique, la coenzyme B 12, la lipoyllysine, le 11-cis-rétinal, et le 1,25-dihydroxycholécalciférol. Le terme vitamine inclut également la choline, la carnitine, et les alpha, beta, et gamma carotènes.

Tel qu'utilisé ici, le terme "minéral" fait référence aux substances inorganiques, aux métaux, et similaires nécessaires dans l'alimentation humaine. Ainsi, le terme "minéral" inclut notamment , le calcium, le fer, le zinc, le sélénium, le cuivre, l'iode, le magnésium, le phosphore, le chrome et similaires, et leurs mélanges.

Le terme " supplément nutritionnel" tel qu'utilisé ici, signifie une substance qui a un effet nutritionnel appréciable lorsqu'il est administré en petites quantités. Les suppléments nutritionnels comprennent notamment des substances telles que le pollen d'abeilles, le son, les germes de blé, le varech, l'huile de foie de morue, le ginseng, et les huiles de poissons, les acides aminés, les protéines et leurs mélanges. Les suppléments nutritionnels peuvent incorporer des vitamines et sels minéraux.

Les quantités de principe actif incorporées dans chaque comprimé peuvent être choisies selon des principes connus en pharmacie. Généralement on incorpore une quantité efficace de principe actif.

L'agent alcalin et l'agent acide sont choisis parmi ceux qui sont pharmaceutiquement acceptables de telle sorte qu'en présence d'eau ils permettent la libération d'un gaz. L'objectif du mélange effervescent est ici multiple :
- libération d'un gaz pharmaceutiquement acceptable facilitant la désagrégation rapide du comprimé au contact de la salive et conférant les propriétés organoleptiques recherchées (masquage de goût, sensation de chatouillement,...)
- Induction d'un micro pH buccal favorisant les propriétés organoleptiques recherchées en influençant par exemple la solubilité du principe actif de façon à diminuer sa perception dans la cavité buccale ou en induisant un goût acidulé agréable.

Le volume de gaz dégagé est compris entre 2 et 60 cm³ et préférentiellement entre 5 et 40 cm³

Au delà de 60 cm³, le volume induit est trop important et devient désagréable au niveau de la cavité buccale. Cette caractéristique différencie la présente invention des comprimés effervescents classiques destinés à être dissous dans un verre d'eau, pour lesquels le volume de gaz est en général plus important, 100 à 300 cm³ dépendant du format et de la composition du comprimé.

L'agent acide est un composé donneur de protons qui peut réagir avec un agent alcalin pour former un gaz qui provoque l'effervescence du liquide dans lequel ce gaz est libéré.

L'agent acide peut être constitué par tout acide minéral ou organique, sous forme d'acide libre, d'anhydride d'acide ou de sel d'acide.

Selon un mode de réalisation avantageux, l'acide sous forme solide à la température ambiante présente un pH inférieur ou égal à 4,5.

Cet acide est choisi dans le groupe comprenant notamment l'acide tartrique, l'acide citrique, l'acide maléique, l'acide fumarique, l'acide malique, l'acide adipique, l'acide succinique, l'acide lactique, l'acide glycolique, les alpha hydroxy acides, l'acide ascorbique et les acides aminés, ainsi que les sels et dérivés de ces acides.

L'agent alcalin est constitué par un composé capable de générer un gaz par réaction avec un composé donneur de proton. Le gaz formé est le dioxyde de carbone, l'oxygène ou le dioxyde de chlore ou tout autre type de gaz biocompatible.

Selon un mode de réalisation avantageux, l'agent alcalin est choisi dans le groupe comprenant le carbonate, de potassium, de lithium, de sodium, de calcium, d'ammonium ou le carbonate de L-lysine, le carbonate d'arginine, le carbonate de glycine sodique, les carbonates sodiques d'acides aminés, le perborate de sodium anhydre, le perborate effervescent, le monohydrate de perborate sodique, le percarbonate de sodium, le dichloroisocyanurate de sodium, l'hypochlorite de sodium, l'hypochlorite de calcium, et leurs mélanges.

Dans le cadre de la présente invention, on entend par carbonate, indifféremment les carbonates, les sesquicarbonates et les hydrogénocarbonates.

Les quantités respectives d'agent acide et d'agent alcalin sont adaptées pour que la réaction entre l'agent alcalin et les protons libérés par l'acide permette la génération d'une quantité suffisante de gaz de façon à obtenir une effervescence satisfaisante.

Selon un mode de réalisation avantageux et notamment lorsque le principe actif a un goût désagréable, on met en oeuvre un excès d'agent alcalin ou d'agent acide de façon à maintenir autour du principe actif un environnement de pH déterminé, environnement dans lequel le goût du principe actif est atténué.

Selon un mode de réalisation avantageux, l'agent acide et l'agent alcalin sont mis en oeuvre dans les granules effervescents sous forme pulvérulente ou granulaire.

Ce mélange effervescent peut selon un mode de réalisation avantageux, être complété par un agent déshydratant interne dont le rôle est de stabiliser le produit, notamment vis à vis de la teneur en eau contenue dans le comprimé afin d'éviter un démarrage de réaction d'effervescence au cours de la conservation du produit. Les agents déshydratants internes classiquement utilisés sont le carbonate monosodique, le dicitrate trimagnesien anhydre, ou tout autre, sel pharmaceutiquement compatible, avide d'eau.

Cet agent déshydratant peut être introduit directement dans le granulé effervescent, ou bien il peut être ajouté en phase externe aux autres excipients du comprimé par mélange de poudre, ou bien, il peut être ajouté de façon partagée à la fois au granule effervescent et au mélange d'excipients du comprimé.

Le liant extrudable à chaud est un liant qui est suffisamment rigide à la température et à la pression ambiantes mais qui peut être déformé ou qui peut former une matière semi-liquide sous pression ou à température élevées.

Selon un mode de réalisation particulier, ce liant présente une température de fusion ou de ramollissement inférieure à 150°C.

Néanmoins, il est possible d'utiliser des liants présentant une température de fusion ou de ramollissement supérieure à 150°C dans la mesure où ils sont utilisés en présence d'un agent plastifiant. Les plastifiants utilisables dans la présente invention peuvent être choisis dans le groupe comprenant notamment les polymères de bas poids moléculaire, les oligomères, les copolymères, les huiles, les petites molécules organiques, les polyols de bas poids moléculaire ayant des groupes hydroxyls aliphatiques, les plastifiants de type ester, les éthers de glycol, le poly(propylène glycol), les polymères multiséquencés, les polymères monoséquencés, le poly(éthylène glycol) de bas poids moléculaire, les plastifiants de type citrate ester, la triacétine, le propylène glycol et la glycérine.

Ces plastifiants peuvent également être l'éthylène glycol, le 1,2-butylène glycol, le 2,3-butylène glycol, le styrène glycol, le diéthylène glycol, le thriéthylène glycol, le tétraéthylène glycol et d'autres composés de type poly(éthylène glycol), le monopropylène glycol monoisopropyl éther, le propylène glycol monoéthyl éther, le sorbitol lactate, le butyl lactate, l'éthylglycolate, le dibutylsébacate, l'acétyltributylcitrate, le triéthyl citrate, l'acétyl triéthyl citrate, le tributyl citrate et l'allyl glycolate, et leurs mélanges.

Il est également possible d'introduire, lorsque cela est nécessaire, un agent antioxydant pharmaceutique compatible choisi dans les groupes des antioxygènes, agents réducteurs ou agent synergiques des antioxygènes. Il s'agit notamment pour le premier groupe des dérivés de tocophérol, dans le cas des agents réducteurs, de l'acide ascorbique, et dans le troisième groupe des acides citrique, tartrique et de la lécithine.

Le liant extrudable peut être choisi dans le groupe comprenant notamment la gomme tragacanth, la gélatine, l'amidon, les dérivés cellulosiques tels que la carboxyméthylcellulose sodique, la méthyl cellulose, les dérivés de povidone, les dérivés de l'acide méthacrylique, les acides alginiques et leurs sels, les polyéthylène glycols, la gomme guar, les polysaccharides, le sucrostéarate, les poloxamers (PLURONIC F68, PLURONIC F127), le collagène, l'albumine, la gélatine, et leurs mélanges.

D'autres liants sont choisis dans le groupe comprennent notamment le propylène glycol, le copolymère polyoxyéthylène-polypropylène, le polyéthylène ester, le polyéthylène sorbitan ester, l'oxyde de polyéthylène et similaires.

Selon un mode de réalisation particulièrement avantageux , le liant est un polyéthylène glycol, ayant de préférence une masse moléculaire de 1000 à 8000 Da.

Le liant peut être utilisé sous n'importe quelle forme, notamment sous forme de poudre, de granules, de copeaux, ou à l'état fondu.

Selon un mode de réalisation avantageux, la quantité de liant utilisé dans les granules effervescents est inférieure à 60%, de préférence comprise entre 3 et 8% en poids par rapport au poids des granules effervescents.

Dans le cas où le principe actif est sous forme acide, le principe actif peut lui-même faire office de donneur de protons pour générer l'effervescence en présence de l'agent alcalin.

Le principe actif est sous forme de microparticules, de microgranules ou sous forme de particules constituées d'un noyau neutre sur lequel sont déposés les cristaux de principe actif.

Le polymère d'enrobage du principe actif est choisi de façon avantageuse dans le groupe comprenant les polymères cellulosiques, les polymères acryliques, les polymères vinyliques, les cires et dérivés et leurs mélanges.

Parmi les polymères cellulosiques, on choisira avantageusement l'éthycellulose, l'hydroxypropylcellulose (HPC) et l'hydroxypropylméthylcellulose (HPMC), seule ou en mélange, l'acétophtalate de cellulose.

Parmi les polymères acryliques, on choisira avantageusement le copolymère amonio-métacrylate (Eudragit^{®} RL et RS), le polyacrylate (Eudragit^{®} NE) et le polymétacrylate (notamment connu sous l'appellation Eudragit^{®} E), Eudragit^{®} étant une marque déposée par RÖHM & HAAS.

Le polymère d'enrobage est présent dans des proportions pouvant aller jusqu'à 50 % en poids, de préférence de 10 % à 35 % en poids par rapport au poids des particules enrobées de principe actif.

Selon un autre mode de réalisation avantageux, l'enrobage de masquage de goût est un enrobage entérique.

Les comprimés conformes à l'invention comportent un mélange d'excipients comprenant:
- un agent de désintégration,
- un agent soluble diluant à propriétés liantes,
- un lubrifiant, et
- éventuellement un agent déshydratant interne, un agent gonflant, un agent perméabilisant, des édulcorants, des arômes et des colorants.

Selon un mode de réalisation avantageux, le ratio excipient / principe actif enrobé ou non est de 0,4 à 6 , de préférence de 1 à 4.

L'agent de désintégration est choisi dans le groupe comprenant notamment la carboxyméthylcellulose sodique réticulée désignée dans le métier par le terme croscarmellose, la crospovidone et leurs mélanges.

L'agent soluble diluant à propriétés liantes est de préférence constitué par un polyol ayant moins de 13 atomes de carbone et se présentant soit sous la forme du produit directement compressible dont le diamètre moyen des particules est de 100 à 500 µm, soit sous la forme d'une poudre dont le diamètre moyen des particules est inférieur à 100 µm, ce polyol étant de préférence choisi dans le groupe comprenant le mannitol, le lactitol, l'erythritol et dérivés, le xylitol, le sorbitol et le maltitol, étant entendu que le sorbitol ne peut pas être utilisé seul et que, dans le cas où l'agent soluble diluant à propriétés liantes serait unique, il est utilisé sous la forme du produit directement compressible alors que, dans le cas où il y aurait au moins deux agents solubles diluants à propriétés liantes, l'un est présent sous la forme directement compressible et l'autre sous la forme poudre, le polyol pouvant être le même, les proportions de polyol directement compressible et de polyol poudre étant de 99/1 à 20/80, de préférence de 80/20 à 20/80.

Les proportions respectives d'agent de désintégration et d'agent soluble retenues pour la constitution de l'excipient sont, par rapport à la masse du comprimé, de 1 à 15 % , de préférence de 2 à 7% en poids pour le premier et de 30 à 85%, de préférence de 40 à 70% en poids pour le second.

Le lubrifiant est choisi dans le groupe comprenant notamment le stéarate de magnésium, l'acide stéarique, le stéarylfumarate de sodium, le polyoxyethylèneglycol micronisé (Macrogol 6000 micronisé), la leucine, le talc, le benzoate de sodium et leurs mélanges.

La quantité d'agent lubrifiant est de 0,2 à 20 pour mille (poids de l'agent lubrifiant/poids total du comprimé), de préférence de 5 à 10 pour mille.

L'agent lubrifiant peut être réparti au sein de l'excipient ; selon un mode de réalisation avantageux, la totalité de l'agent lubrifiant peut être répartie à la surface du comprimé (demande de brevet WO 00/51568).

L'agent lubrifiant peut éventuellement être présent dans les granules effervescents.

Comme agent perméabilisant on utilise avantageusement un composé choisi dans le groupe comprenant notamment des silices ayant une grande affinité pour les solvants aqueux, telles que la silice précipitée plus connue sous le nom de marque Syloïd^{®}, les maltodextrines, les β-cyclodextrines et leurs mélanges.

L'agent perméabilisant permet la création d'un réseau hydrophile qui facilite la pénétration de la salive et contribue ainsi à une meilleure désagrégation du comprimé.

La proportion d'agent perméabilisant par rapport à la masse du comprimé est de 0% à 10% en poids.

L'édulcorant peut être choisi dans le groupe comprenant notamment l'aspartame, l'acésulfame de potassium, le saccharinate de sodium, la néohespéridine dihydrochalcone, le sucralose, le monoammonium glycyrrhizinate et leurs mélanges.

Les arômes et colorants sont ceux utilisés habituellement en pharmacie pour la préparation de comprimés.

Dans un mode de réalisation particulièrement adapté aux principes actifs présentant un goût très désagréable, le comprimé comprend:
- 15% à 70%, de préférence de 20% à 50%, de granules effervescents contenant un principe actif enrobé,
- 1% à 15%, de préférence de 2% à 7%, d'agent de désintégration,
- 0% à 30%, de préférence de 2% à 15%, d'agent déshydratant,
- jusqu'à 1,5%, de préférence de 0,25% à 1%, d'agent lubrifiant,
- 0 à 10%, de préférence de 0,5% à 5%, d'agent perméabilisant,
- 0 à 3,5%, de préférence de 0,5% à 3%, de colorant
- 0 à 3%, de préférence de 0,5% à 2,5%, d'arôme,
- agent soluble diluant qsp 100%,
les pourcentages étant calculés en poids par rapport au poids total du comprimé.

Les comprimés selon l'invention permettent d'obtenir des temps de délitement dans la bouche, sous l'action de la salive et en l'absence de toute action de mastication, inférieurs à 60 secondes et de préférence inférieurs à 40 secondes. Ce temps de délitement correspond à la durée qui sépare, d'une part, le moment de la mise en place du comprimé dans la bouche au contact de la salive et, d'autre part, le moment de la déglutition de la suspension résultant de la désagrégation sans mastication du comprimé au contact de la salive.

Ces comprimés présentent une dureté et une abrasion suffisantes pour résister aux diverses manipulations nécessaires à leur conditionnement et à leur stockage.

Ils ont des qualités organoleptiques satisfaisantes.

L'invention pourra être encore mieux comprise à l'aide des exemples qui suivent et qui ne sont pas limitatifs mais relatifs à des modes de réalisation avantageux de l'invention.

### EXEMPLE 1: Granules effervescents ne contenant pas de principe actif préparés selon un premier mode opératoire:

On prépare 10 types de granules effervescents, identifiés respectivement par les lettres A à J dont les compositions sont données ci-après

La préparation de ces granules effervescents est réalisée sous atmosphère contrôlée en humidité, avec séchage éventuel avant mise en oeuvre des composants trop humides. Les différents constituants identifiés ci-après, peuvent être préalablement mélangés dans un mélangeur externe ou directement dans l'extrudeuse à chaud.

Dans l'extrudeuse à chaud, .le mélange est porté à une température au plus égale à environ 120°C à une vitesse et pendant un temps suffisants pour faire fondre ou ramollir le liant.

On récupère l'extrudat que l'on coupe ou que l'on broie.

Les granules effervescents ainsi préparés sont alors tamisés avec un tamis de 710 microns d'ouverture de mailles, puis stockés dans une atmosphère de faible humidité.

| **Granules A** | |
|---|---|
| **Constituants** | **Quantités** (% en poids) |
| NaHCO₃ | 52 |
| Acide citrique | 14 |
| Acide tartrique | 28 |
| PEG 1000 | 6 |

| **Granules B** | |
|---|---|
| **Constituants** | **Quantités** (% en poids) |
| NaHCO₃ | 55 |
| Acide citrique | 13,5 |
| Acide tartrique | 24 |
| PEG 4000 | 7,5 |

| **Granules C** | |
|---|---|
| **Constituants** | **Quantités** (% en poids) |
| Carbonate de glycine sodique | 58 |
| Acide citrique | 15 |
| Acide tartrique | 21 |
| Pluronic F68 | 6 |

| **Granules D** | |
|---|---|
| **Constituants** | **Quantités** (% en poids) |
| NaHCO₃ | 54 |
| Acide citrique | 16 |
| Acide tartrique | 24 |
| PEG 20000 | 3 |
| PEG 400 | 3 |

| **Granules E** | |
|---|---|
| **Constituants** | **Quantités** (% en poids) |
| NaHCO₃ | 50 |
| Acide citrique | 14 |
| Acide tartrique | 28 |
| PEG 8000 | 8 |

| **Granules F** | |
|---|---|
| **Constituants** | **Quantités** (% en poids) |
| KHCO₃ | 62 |
| Acide fumarique | 5 |
| Acide citrique | 8 |
| Acide tartrique | 18 |
| PEG 6000 | 7 |

| **Granules G** | |
|---|---|
| **Constituants** | **Quantités** (% en poids) |
| NaHCO₃ | 55 |
| NaH₂PO₄ | 37,5 |
| Pluronic F127 | 7,5 |

| **Granules H** | |
|---|---|
| **Constituants** | **Quantités** (% en poids) |
| NaHCO₃ | 54 |
| Acide fumarique | 3 |
| Acide maléique | 5 |
| Acide citrique | 13 |
| Acide tartrique | 18 |
| PEG 3350 | 6 |
| Pluronic F68 | 4 |

| **Granules I** | |
|---|---|
| **Constituants** | **Quantités** (% en poids) |
| NaHCO₃ | 56 |
| Acide citrique | 37 |
| Cétyl alcool | 4 |
| Stéaryl alcool | 5 |

| **Granules J** | |
|---|---|
| **Constituants** | **Quantités** (% en poids) |
| NaHCO₃ | 51 |
| Acide citrique | 34 |
| Xylitol | 15 |

### Exemple 2: Préparation de comprimés comprenant des granules effervescents qui eux mêmes comprennent du Methylprednisolone enrobé. (dosage: 5mg)

On prépare des granules effervescents contenant du Methylprednisolone comme principe actif.

Ces granules sont préparés comme dans l'exemple 1, excepté que le methylprednisolone est ajouté au mélange pulvérulent. On obtient ainsi des granules effervescents présentant la composition centésimale suivante:

| | |
|---|---|
| Granules enrobés de methylprednisolone | 9,825 % |
| Bicarbonate de sodium | 45 % |
| Acide citrique, anhydre | 35 % |
| Cellulose microcristalline | 8,175 % |
| Stéarate de saccharose | 2 % |

Les granules enrobés de Methylprednisolone mis en oeuvre dans les granules effervescents présentent la composition centésimale suivante:

| | |
|---|---|
| Methylprednisolone | 80 % |
| Silice précipitée | 4 % |
| Eudragit NE30D | 16 % |

Les granules effervescents contenant les granules enrobés de methylprednisolone sont mis en oeuvre dans des comprimés conformes à l'invention dosés à 5 mg de Methylprednisolone et qui présentent la composition centésimale suivante:

| Ingrédients | Composition centésimale |
|---|---|
| Granules effervescents de Methyprednisolone enrobé | 50% |
| Mannitol granulé | 20% |
| Mannitol poudre | 20% |
| Croscarmellose | 4% |
| Silice précipitée | 2% |
| Aspartam | 3% |
| Arôme orange | 0,9% |
| Stéarate de magnésium | 0,1% |

Les différents ingrédients ci-dessus sont mélangés et le mélange ainsi obtenu est comprimé à l'aide d'une comprimeuse rotative.
Le diamètre des comprimés est de 8 mm.
Les comprimés obtenus sont des comprimés de 200mg et qui présentent une dureté comprise entre 15 et 40 Newton.
Le temps de délitement dans la bouche est inférieur à 60 secondes. Ce temps est mesuré comme dans l'exemple 2.
La sensation en bouche de ces comprimés est apparue satisfaisante.

### Exemple 3: Préparation de comprimés comprenant des granules effervescents qui eux mêmes comprennent du Methylprednisolone enrobé. (dosage: 20mg)

On prépare des granules effervescents contenant du Methylprednisolone comme principe actif.
Ces granules sont préparés comme dans l'exemple 1, excepté que le methylprednisolone est ajouté au mélange pulvérulent. On obtient ainsi des granules effervescents présentant la composition centésimale suivante:

| Constituants | Composition centésimale |
|---|---|
| Granules enrobés de Méthylprednisolone | 29,475 % |
| Bicarbonate de sodium | 22,4 % |
| Acide citrique, anhydre | 38,125 % |
| Cellulose microcristalline | 8 % |
| Stéarate de saccharose | 2 % |

Les granules enrobés de Methylprednisolone mis en oeuvre dans les granules effervescents présentent la composition centésimale suivante:

| | |
|---|---|
| Methylprednisolone | 80 % |
| Silice précipitée | 4 % |
| Eudragit NE30D | 16 % |

Les granules effervescents contenant les granules enrobés de methylprednisolone sont mis en oeuvre dans des comprimés conformes à l'invention dosés à 20 mg de Methylprednisolone et qui présentent la composition centésimale suivante:

| | |
|---|---|
| Granules effervescents de Methyprednisolone enrobé | 41,67% |
| Mannitol granulé | 24,165% |
| Mannitol poudre | 24,165% |
| Croscarmellose | 4% |
| Silice précipitée | 2% |
| Aspartam | 3% |
| Arôme orange | 0,9% |
| Stéarate de magnésium | 0,1% |

Les différents ingrédients ci-dessus sont mélangés et le mélange ainsi obtenu est comprimé à l'aide d'une comprimeuse rotative.
Le diamètre des comprimés est 10 mm.
Les comprimés obtenus sont des comprimés de 300mg et présentent une dureté comprise entre 15 et 40 Newton.
Le temps de délitement dans la bouche est inférieur à 60 secondes. Ce temps est mesuré comme dans l'exemple 2.
La sensation en bouche de ces comprimés est apparue satisfaisante.

## Revendications

1. Comprimé effervescent orodispersible comprenant:
- un mélange d'excipients comprenant au moins un agent de désintégration choisi parmi la croscarmellose, la crospovidone et leur mélange, un agent soluble diluant, un agent lubrifiant et éventuellement un agent déshydratant interne, un agent gonflant, un agent perméabilisant, des édulcorants, des arômes et des colorants,
- et des granules effervescents à base d'au moins un principe actif enrobé et d'un mélange constitué d'un agent acide, d'un liant extrudable à chaud et d'un agent alcalin et éventuellement d'un agent déshydratant et d'un lubrifiant,
lesdits granules étant préparés par extrusion à chaud en l'absence d'eau et de solvants,
et ledit comprimé se désagrégeant dans la cavité buccale au contact de la salive en moins de 60 secondes et préférentiellement en moins de 40 secondes.

2. Comprimé selon la revendication 1
**caractérisé par le fait que** le principe actif est enrobé à l'aide d'un enrobage lui conférant des propriétés de masquage de goût, de stabilisation, de gastro-résistance ou de libération modifiée.

3. Comprimé selon l'une quelconque des revendications 1 à 2 dans lequel le ratio mélange d'excipients / principe actif est de 0,4 à 6 , de préférence de 1 à 4.

4. Comprimé selon l'une quelconque des revendications 1 à 3, **caractérisé par le fait qu'**il comprend :
- 15% à 70%, de préférence de 20% à 50%, de granules effervescents contenant un principe actif enrobé,
- 1% à 15%, de préférence de 2% à 7%, d'agent de désintégration,
- 0% à 30%, de préférence de 2% à 15%, d'agent déshydratant,
- jusqu'à 1,5%, de préférence de 0,25% à 1%, d'agent lubrifiant,
- 0 à 10%, de préférence de 0,5% à 5%, d'agent perméabilisant,
- 0 à 3,5%, de préférence de 0,5% à 3%, de colorant,
- 0 à 3%, de préférence de 0,5% à 2,5%, d'arôme,
- agent soluble diluant qsp 100%,
les pourcentages étant calculés en poids par rapport au poids total du comprimé.

## Claims

1. An orodispersible effervescent tablet comprising:
- a mixture of excipients comprising at least one disintegrant chosen among croscarmellose, crospovidone and their mixture, a diluent soluble agent, a lubricant and optionally an internal dehydrating agent, a swelling agent, a permeabilizing agent, sweeteners, flavorings and colorants,
- and effervescent granules based on at least one coated active principle and a mixture constituted by an acidic agent, a heat-extrudable binder and an alkaline agent and optionally a dehydrating agent and a lubricant,
said granules being prepared by heat extrusion in the absence of water and solvents,
and said tablet undergoing disaggregation in the buccal cavity in contact with saliva in less than 60 seconds and preferably in less than 40 seconds.

2. A tablet according to claim 1, **characterized in that** the active principle is coated with a coating which gives it properties of taste masking, stabilization, gastric fluid resistance or modified release.

3. A tablet according to any one of claims 1 to 2, **characterized in that** the excipients mixture/active principle ratio is from 0.4 to 6, preferably from 1 to 4.

4. A tablet according to any one of claims 1 to 3, **characterized in that** it comprises:
- 15% to 70%, preferably from 20% to 50%, of effervescent granules comprising a coated active principle,
- 1% to 15%, preferably from 2% to 7%, of disintegrant,
- 0% to 30%, preferably from 2% to 15%, of dehydrating agent,
- up to 1.5%, preferably from 0.25% to 1%, of lubricant,
- 0 to 10%, preferably from 0.5% to 5%, of permeabilizing agent,
- 0 to 3.5%, preferably from 0.5% to 3%, of colorant,
- 0 to 3%, preferably from 0.5% to 2.5%, of flavoring,
- diluent soluble agent qs 100%,
the percentages being calculated by weight relative to the total weight of the tablet.

## Patentansprüche

1. Orodispersible Brausetablette umfassend:
- eine Mischung von Hilfsstoffen umfassend mindestens ein Auflösungsmittel, das unter Croscarmellose, Crospovidon und einer Mischung derselben gewählt wird, ein lösliches Verdünnungsmittel, ein Schmiermittel, und eventuell ein internes Entwässerungsmittel, ein Quellmittel, ein Durchlässigkeitsmittel, Süßungsmittel, Aromen und Färbemittel, und
- Brausegranulat basierend auf mindestens einem überzogenen Wirkstoff und einer Mischung, die aus einem Säuremittel, einem warmstrangpressbaren Bindemittel und einem alkalischen Mittel und eventuell einem Entwässerungsmittel und einem Schmiermittel besteht wobei
das Granulat durch Warmstrangpressen in Abwesenheit von Wasser und von Lösungsmitteln hergestellt wird, und wobei
sich die Tablette bei Berührung mit dem Speichel in der Mundhöhle in weniger als 60 Sekunden, vorzugsweise in weniger als 40 Sekunden, zersetzt.

2. Tablette nach Anspruch 1, **dadurch gekennzeichnet, dass** der Wirkstoff mittels eines Überzugsmittels überzogen ist, das dem Wirkstoff folgende Eigenschaften verleiht: Überdeckung des Geschmacks, Stabilisierung, Magenresistenz oder modifizierte Freisetzung.

3. Tablette nach irgendeinem der Ansprüche 1 bis 2, wobei das Mischungsverhältnis Hilfsstoffe / Wirkstoff 0,4 bis 6, vorzugsweise 1 bis 4, beträgt.

4. Tablette nach irgendeinem der Ansprüche 1 bis 3, **dadurch gekennzeichnet, dass** die Tablette Folgendes umfasst:
- 15% bis 70%, vorzugsweise von 20% bis 50% Brausegranulat, das einen überzogenen Wirkstoff enthält,
- 1% bis 15%, vorzugsweise von 2% bis 7% Auflösungsmittel,
- 0% bis 30%, vorzugsweise von 2% bis 15% Entwässerungsmittel,
- bis zu 1,5%, vorzugsweise von 0,25% bis 1% Schmiermittel,
- 0 bis 10%, vorzugsweise von 0,5% bis 5% Durchlässigkeitsmittel,
- 0 bis 3,5%, vorzugsweise 0,5% bis 3% Färbemittel,
- 0 bis 3%, vorzugsweise 0,5% bis 2,5% Aroma,
- lösliches Verdünnungsmittel qsp 100 %,
wobei die Prozentangaben in Gewicht im Verhältnis zu dem Gesamtgewicht der Tablette berechnet sind.
